# FASCICULE DE BREVET EUROPEEN

(11) **EP 2 168 066 B1**
(45) Date de publication et mention de la délivrance du brevet: **13.11.2019**
(21) Numéro de dépôt: 07803751.2
(22) Date de dépôt: 14.06.2007
(51) Int. Cl.: G16H 50/30

(54) **METHODE DE DETERMINATION DE SCORES DE RISQUES**
VERFAHREN ZUR BESTIMMUNG VON RISIKOBEWERTUNGEN
METHOD FOR DETERMINING RISK SCORES

(43) Date de publication de la demande: 31.03.2010
(73) Titulaire: PREDILIFE, 94805 Villejuif Cedex (FR)
(72) Inventeur: RAGUSA, Stéphane, 75007 Paris (FR)
(74) Mandataire: Vial, Lionel
(86) Numéro de dépôt international: PCT/FR2007/000990
(87) Numéro de publication internationale: WO 2008/152212

(56) Documents cités:
- WO-A-2007/050186

## Description

### CONTEXTE DE L'INVENTION

La présente invention concerne une méthode de détermination de scores de risques de pathologies pour un individu donné à partir d'une cohorte prospective. Une telle méthode ne fournit pas un diagnostic médical mais constitue un outil d'assistance au médecin pour estimer les risques de pathologies futures d'un individu encore sain et permettre au médecin d'adapter les traitements préventifs éventuellement nécessaires.

### ETAT DE LA TECHNIQUE

L'évaluation des risques liés à un individu est une approche très fréquente dans les secteurs financiers, tels que les assurances ou les établissements de crédit. Cette approche d'évaluation des risques liés à un individu intéresse également le secteur médical qui voit dans cette évaluation des risques un moyen d'adapter des mesures préventives à chaque individu en fonction de ses scores de risques. Par exemple, un individu présentant un score de risque élevé à tel cancer pourrait se voir appliquer des méthodes de diagnostic plus coûteuses ; et un individu présentant un score de risques élevé d'accident cardiovasculaire peut se voire prescrire des traitements hypolipémiants avant même l'apparition de premiers symptômes et sans niveau particulièrement élevé de chacun des facteurs de risque. Cette évaluation du risque cardiovasculaire global fait d'ailleurs partie des recommandations internationales. A titre d'exemple, la demande WO2007/050186 décrit une méthode générale de détermination simultanée d'une pluralité- de risques médicaux à l'aide d'un modèle de détermination de risque médicaux.

De telles méthodes d'évaluation des risques médicaux sont délicates à réaliser. En effet, si dans les secteurs des assurances ou des établissements de crédits les méthodes statistiques pour évaluer les risques d'un nouvel individu sont assez simples étant donnée l'abondance des données, il n'en est pas de même dans le secteur médical. Les bases de données médicales sont rares, coûteuses, de taille souvent réduite rendant nécessaires des méthodes plus complexes pour limiter au maximum la perte d'information au cours du traitement statistique. D'autre part, la rareté des données au plan mondial nécessite de pouvoir facilement exporter des résultats acquis dans un pays vers d'autres pays tout en respectant au maximum les spécificités de ces derniers.

Aujourd'hui, plusieurs types de données sont théoriquement disponibles pour évaluer des scores de risques de pathologies pour un individu encore sain. Ces données médicales peuvent se trouver dans les fichiers de compagnies d'assurance ; ces données sont abondantes mais peuvent être biaisées par un manque d'objectivité des assurés. D'autres données médicales sont disponibles dans les fichiers des hôpitaux ; ces données concernent la population malade et sont donc partielles. Il existe aussi des données fournies par des enquêtes épidémiologiques dites « cas témoin » où l'on interroge rétrospectivement des malades et des non malades sur leur mode de vie antérieur ; on se heurte souvent à une mémoire sélective qui tend à minorer certains facteurs de risque comme le tabac. Il existe aussi des enquêtes épidémiologiques dites « prospectives » où l'on bâtit une «cohorte prospective », c'est-à-dire que l'on considère toute une population pour laquelle on enregistre des paramètres individuels (mode de vie, alimentation ...) et que l'on suit pendant de longues années pour répertorier les pathologies développées.

Les cohortes prospectives sont aujourd'hui les outils les plus adaptés à l'estimation de scores de risques de pathologies. Néanmoins ces cohortes sont assez rares dans le monde et il est nécessaire de pouvoir exporter les résultats acquis sur une cohorte d'un pays vers des populations d'autres pays.

Pour un nouvel individu, une estimation de son risque relatif individuel par rapport à un individu de même âge dans la cohorte initiale, multiplié par l'incidence moyenne à cet âge dans la population finale de cet individu permet d'estimer correctement son risque de développer telle pathologie

Une telle méthode d'estimation de scores de risques basée sur une cohorte prospective nécessite de disposer d'une telle cohorte et surtout que cette cohorte contienne un nombre suffisant d'individus pour voir apparaître suffisamment de malades. En effet, si l'on considère une cohorte prospective de 10 000 individus, le nombre d'individus ayant développé une pathologie au bout de 10 ans peut être de l'ordre de quelques centaines pour les maladies cardiovasculaires ou les cancers du sein, du poumon ou du côlon et de l'ordre de quelques dizaines pour les autres pathologies moins prévalentes. Des cohortes prospectives de 100 000 individus sont donc plus adaptées mais de telles cohortes n'existent qu'à quelques exemplaires dans le monde et certaines ont peu de paramètres enregistrés.

Les médecins disposent aujourd'hui principalement de modèles d'estimation de scores de risques pour deux grandes pathologies : les risques cardiovasculaires avec les scores issus de la cohorte de Framingham et le risque de cancer du sein avec le modèle de Gail issu d'une enquête cas témoins. Ces deux scores sont américains, estimés à partir de populations des Etats-Unis.

Le modèle de Gail est présenté dans la publication de Gail MH et al., « Projecting individualized probabilities of developing breast cancer for white females who are being examined annually », J Nat Cancer Inst 1989 Dec 20;81(24):1879-86 ; ce modèle est utilisé pour évaluer un risque avant une mastectomie éventuelle ou comme un critère d'indentification de patientes avant leur inclusion dans un essai clinique et comme une aide au choix d'une prescription thérapeutique. Les applications du modèle de Gail sont décrites par exemple dans les publications de Freedman AN et al., « Estimâtes of the number of US women who could benefit from tamoxifen for breast cancer chemoprevention », J Natl Cancer Inst. 2003 Apr 2;95(7):526-32, et de Vogel VG « The study of tamoxifen and raloxifene: preliminary enrollment data from a randomized breast cancer risk réduction trial », Clin Breast Cancer. 2002 Jun;3(2):153-9.

Le modèle de Framingham est présenté dans les publications de Anderson KM et al., « Cardiovascular disease risk profiles », Am Heart J. 1991 Jan;121(1 Pt 2):293-8. et de Wilson PWF et al., « Prédiction of coronary heart disease using risk factor categories », Circulation. 1998 May 12;97(18):1837-47. Ce modèle de Framingham est couramment utilisé par les médecins pour adapter une prescription thérapeutique en cardiologie et cibler la prévention. Des outils logiciels réalisés par des laboratoires ou des institutions sont diffusés, mettant en œuvre l'algorithme de Framingham; le médecin saisit à l'écran des données relatives à des paramètres personnels de l'individu à traiter, tels que le sexe, l'age, le poids, le taux de cholestérol... et le logiciel fournit un score de risques représentatif des possibilités que ledit individu soit sujet à une maladie coronarienne, un accident vasculaire cérébral...

Les modèles de Gail et de Framingham sont aujourd'hui utilisés pour définir des scores de risques sur des populations différentes de la population représentée par la cohorte prospective initiale. Si la population finale dispose d'une cohorte, la méthodologie peut être reprise et des paramètres réestimés sur la population finale (ex Decarli A, et al. J Natl Cancer Inst. 2006 Dec 6;98(23):1686-93) mais malheureusement, la majorité des pays ne disposent pas de cohortes prospectives en propre. Or, on a constaté que lorsqu'un tel modèle est exporté de manière brute vers une population différente de la population de départ, les scores de risques résultant surestiment ou sous estiment très souvent les risques réels. C'est ce qui a été établi par exemple dans la publication de Empana JP, et al. Eur Heart J. 2003 Nov;24(21):1903-11.

Pour corriger ce biais, on peut utiliser des données d'une cohorte plus représentative de la population de l'individu à traiter. Mais en l'absence de telles cohortes plus représentatives, les médecins sont souvent réduits à diviser ou multiplier mentalement le risque obtenu.

Il faut donc une méthode permettant de réaliser un score de risque qui puisse être facilement exporté vers d'autres pays où l'on ne dispose pas forcément d'une cohorte prospective, mais seulement de l'incidence de la pathologie considérée soit en fonction de l'âge, soit tous âges confondus, données beaucoup plus fréquentes.

Les modèles de Framingham et de Gail ne permettent pas cette exportation flexible. Lorsqu'ils sont utilisés hors des Etats-Unis ils utilisent les mêmes paramètres que pour un américain lorsque la population finale n'a pas de cohorte.

Le modèle de Gail par exemple ne peut utiliser l'incidence du cancer du sein d'un autre pays, car il fait intervenir un risque « de base » , pour un individu hypothétique n'ayant aucun facteur de risque. Or ce risque de base théorique est construit à partir des risques réels observés sur une cohorte. Ainsi, par construction, ce modèle ne peut pas être adapté à une population d'un autre pays pour lequel on ne disposerait que de la simple incidence de cancer du sein en fonction de l'âge. Lorsqu'on veut adapter le modèle de Gail, il est nécessaire d'avoir une cohorte prospective, comme expliqué dans la publication de Decarli A, et al, J Natl Cancer Inst. 2006 Dec 6;98(23):1686-93 qui vise à l'exporter en Italie.

Ce type de méthode est aussi utilisé par ailleurs dans WO 01/66007 qui décrit un procédé d'estimation du risque que présente un sujet de développer une pathologie donnée, durant une période de temps donnée. Selon ce procédé, un profil de base du sujet est tout d'abord dressé de manière à identifier un groupe d'individus auquel appartient le sujet. Ce profil de base peut comprendre des informations démographiques telles que l'age, le genre, l'ethnie ou la région géographique de résidence de l'individu, ou d'autres types d'informations. Un risque de base est ensuite déterminé comme étant le risque hypothétique que le sujet (sans aucun facteur de risque) développe la pathologie considérée durant la période de temps considérée. Ce risque de base est déterminé statistiquement à partir du groupe d'individus correspondant au profil de base du sujet.

Des facteurs de risques supplémentaires pour le sujet de développer la pathologie sont ensuite déterminés à partir d'une étude statistique portant sur un groupe d'individus dont le sujet peut ne pas faire partie. Ces risques supplémentaires peuvent concerner les antécédents familiaux ou l'hygiène de vie du sujet. Un profil épidémiologique du sujet est alors dressé, qui regroupe des valeurs de risques supplémentaires pour le sujet. Le risque de base est alors ajusté en fonction du profil épidémiologique du sujet de manière à générer un risque estimé pour le sujet de développer la pathologie donnée durant la période de temps donnée.

Ce procédé nécessite la création d'un risque de base pour un individu hypothétique n'ayant aucun facteur de risque. Comme avec le modèle de Gail, l'ajustement de ce risque de base en fonction de la simple incidence dans la population cible est impossible.

II existe donc un besoin pour exploiter de manière fiable les données disponibles dans les cohortes existantes sur des populations différentes de la cohorte prospective initiale.

### RESUME DE L'INVENTION

A cet effet, on propose d'utiliser une cohorte prospective et d'estimer un risque relatif pour un individu donné appartenant à une population non représentée par la cohorte, selon les revendications suivantes. Ce risque relatif individuel est alors pondéré par l'incidence de la pathologie dans la population à laquelle appartient cet individu pour aboutir ainsi au risque absolu de cet individu.

On évite ainsi d'appliquer brutalement des équations définies pour une population initiale et qui conduisent à surestimer ou sous estimer les risques pour un individu d'une autre population.

L'invention propose une méthode de détermination de scores de risques de pathologies pour un individu donné appartenant à une population finale, la méthode comprenant les étapes de :
- sélection d'une cohorte prospective à partir d'une population initiale différente de la population finale ;
- estimation d'un module de scores de risques relatif individuel de l'individu à partir de paramètres et de variables de la cohorte prospective, ledit module comprenant au moins un score de risques associé à au moins une variable pathologie ;
- multiplication de chaque score de risques du module relatif individuel par l'incidence de chaque pathologie dans la population finale de l'individu;
dans laquelle l'estimation d'un module de scores de risques relatif individuel comprend pour chaque pathologie les étapes de :
- estimation de risques relatifs (βi) pour chaque paramètre explicatif individuel (vi) de la cohorte et considéré comme explicatif de cette pathologie :
   - construction d'un risque relatif pour chaque paramètre explicatif exprimé comme exp[βi(vi - valeur moyenne de vi)] ;
   - construction du module de scores de risques relatif individuel en multipliant les risques relatifs pour chaque variable pathologie donnée.

Selon une caractéristique, la cohorte prospective comprend :
- une sélection d'individus appartenant à une population initiale ;
- un répertoire de paramètres individuels pour chaque individu ; et
- un répertoire de variables pathologiques pour chaque individu de la cohorte ayant développé des pathologies données.

Selon un mode de réalisation, la cohorte prospective comprend en outre des paramètres thérapeutiques pour chaque individu de la cohorte associés à la prise de traitement thérapeutiques par ledit individu.

Selon un mode de réalisation, les paramètres thérapeutiques comprennent les traitements hormonaux substitutifs et les traitements préventifs de l'ostéoporose.

Selon une caractéristique, le module de scores de risques relatif individuel de l'individu est multiplié par un risque relatif dû à un traitement donné.

L'invention propose aussi un programme d'ordinateur mettant en œuvre la méthode de l'invention. Le programme comprenant une interface permettant la saisie de paramètres individuels associés à un individu donné et la sélection d'au moins une variable pathologique à estimer, ledit programme fournissant un module de scores de risques relatifs individuels associés à ladite variable pathologique pour ledit individu.

Selon un mode de réalisation, l'interface permet la saisie de la population finale à laquelle appartient l'individu donné, ledit programme fournissant un module de score de risque absolu associé à ladite variable pathologique pour ledit individu.

Selon un mode de réalisation, l'interface permet la saisie d'au moins un paramètre thérapeutique associé à la prise d'un traitement thérapeutique, ledit programme fournissant un module de scores de risques matriciel comprenant au moins un score de risques associé à la variable pathologie sélectionnée et au moins un score de risque associé à ladite variable pathologie avec prise dudit traitement thérapeutique.

### EXPOSE DETAILLE DE L'INVENTION

Les caractéristiques et avantages de l'invention apparaîtront à la lecture de la description qui suit de modes de réalisation de l'invention donnés à titre d'exemple.

La description propose une méthode de détermination de scores de risques de pathologies pour un individu donné appartenant à une population finale donnée. Cette méthode utilise une cohorte prospective construite à partir d'une population initiale, qui peut être différente de la population finale de l'individu donné.

La méthode propose tout d'abord de réaliser une estimation d'un module de scores de risques pour au moins une pathologie donnée, ce module de scores de risques étant relatif et individuel pour l'individu donné. Ce module de scores de risques est dit relatif car égal au risque comparé au risque moyen dans la cohorte prospective au même âge ; il est estimé à partir de la cohorte prospective disponible, même si cette cohorte prospective est construite à partir d'une population différente de la population à laquelle l'individu donné appartient. Ce module de scores de risques est dit individuel car il est estimé pour un individu donné à partir de ses paramètres individuels. Le module de scores de risques fournit pour chaque individu une estimation, souvent voisine de 1, de son risque relatif (comparé à la moyenne au même âge) qu'encourt l'individu donné de développer une pathologie donnée.

La méthode propose ensuite de réaliser une multiplication de chaque score de risques du module relatif individuel par l'incidence de la pathologie au même âge dans la population finale de l'individu. Chaque estimation de scores de risques du module sera donc pondérée par un facteur représentatif de la proportion d'individus atteints en une année de la pathologie dans la population finale de l'individu donné aboutissant ainsi à un risque absolu

En effet, l'incidence des pathologies est généralement très hétérogène selon les pays. Le tableau ci-dessous montre l'incidence du cancer du sein en France, aux Etats-Unis et au Japon. L'incidence représente le nombre de femmes ayant développé un cancer du sein pour 100 000 femmes de la même tranche d'age. Les données du tableau pour la France sont consultables sur le site http://www.esculape.com, ou http://www.epidemio.fr. Les données du tableau pour les Etats-Unis sont issues du Programme de surveillance épidémiologique SEER, pour Surveillance Epidemiology and End Results en anglais, consultable sur le site http://www.seer.cancer.gov. Les données du tableau pour le Japon sont tirées du rapport « Progress Report of the Research Group for Population-based Cancer Registration in Japan », 1996 et sont consultables sur le site http://www.ncc.go.jp. De manière générale, les chiffres donnant les incidences moyennes tous âges confondus pour les cancers peuvent aussi être trouvés sur http://www-dep.iarc.fr/, qui dépend de l'OMS.

**Tableau I**

| Age | France | Etats-Unis | Japon |
|---|---|---|---|
| 35-39 | 63 | 55 | 41 |
| 40-44 | 120 | 110 | 75 |
| 45-49 | 187 | 198 | 99 |
| 50-54 | 177 | 263 | 91 |
| 55-59 | 183 | 333 | 68 |
| 60-64 | 211 | 380 | 77 |
| 65-69 | 220 | 430 | 77 |
| 70-74 | 232 | 476 | 71 |
| 75-79 | 220 | 499 | 72 |
| Tous âges | 89 | 137 | 43 |

On constate aisément à partir du tableau I que pour une femme japonaise, un score de risques absolu individuel calculé à partir d'une cohorte de population américaine fournira une estimation de risques de cancer du sein bien trop élevée par rapport aux risques encourus par cette femme japonaise, même si de nombreux paramètres individuels sont similaires entre cette femme japonaise et certaines femmes de la cohorte américaine ayant développées le cancer du sein.

En l'absence de cohorte prospective bâtie à partir de la population japonaise, la méthode décrite ici propose de sélectionner une cohorte prospective bâtie sur une autre population, de déterminer un module de scores de risque relatif individuel et de multiplier les scores de ce module par les incidences des pathologies à considérer.

La méthode proposée comprend donc une première étape de sélection d'une cohorte prospective. La méthode de l'invention peut en effet exploiter une cohorte prospective déjà construite, par exemple une des cohortes construites à partir de la population américaine. La méthode de l'invention peut aussi exploiter une nouvelle cohorte construite par exemple à partir de 100 000 personnes issues de la population française et suivie depuis 15 ans. Une telle cohorte est connue sous la dénomination E3N et les articles parus à son sujet sont consultables sur le site http://www.e3n.net/

Cette cohorte prospective est dite basée sur une population initiale par opposition à la population finale où sera situé l'individu dont on souhaite calculer les risques. Les individus sélectionnés pour constituer la cohorte prospective sont alors suivis sur de nombreuses années et les pathologies qu'ils développent sont répertoriées.

La cohorte prospective intègre des paramètres individuels propres à chaque individu de la population initiale intégré dans la cohorte. Ces paramètres individuels pour chaque individu de la cohorte prospective peuvent comprendre l'âge, le poids, le sexe, des paramètres de comportement nutritif, le tabagisme, le nombre d'enfants, l'âge des premières règles, le taux de cholestérol et d'autres paramètres pertinents. Certains de ces paramètres resterons constants pour un individu donné et d'autres seront variables c'est-à-dire qu'ils seront renseignés en fonction du vieillissement de l'individu surveillé dans la cohorte.

La cohorte inclut aussi des variables pathologiques qui représentent les pathologies développées par les individus de la cohorte. Le nombre d'individus ayant développés une pathologie donnée est donc corrélé à des paramètres individuels.

Il est aussi proposé d'inclure, en plus des paramètres individuels mentionnés ci-dessus, des paramètres thérapeutiques relatifs à des traitements suivis par les individus de la cohorte afin de pouvoir corréler les pathologies développées avec une prise de traitement donné. En particulier, il est proposé de considérer comme un paramètre thérapeutique la prise d'un traitement hormonal substitutif ou d'un traitement préventif de l'ostéoporose donné. La cohorte E3N mentionnée précédemment inclut de tels paramètres. Une telle cohorte prospective permet alors ultérieurement à des médecins d'apprécier les risques relatifs à la prise de tel ou tel traitement par rapport aux risques sans traitement pour une même pathologie. En effet, dans le cas des traitements hormonaux substitutifs (THS) ou destinés à prévenir l'ostéoporose, il est utile d'évaluer individuellement le bénéfice/risque en quantifiant l'impact de la prise de traitement sur chaque risque de pathologies car ces traitements peuvent avoir des effets sur plusieurs risques.

Un nouveau logiciel peut alors être créé utilisant cette nouvelle cohorte prospective qui intègre une base de données incluant des paramètres thérapeutiques associés à la prise de tel ou tel traitement en plus des paramètres individuels « classiques » d'age, de comportement et d'antécédent. La base de données inclut aussi les variables pathologiques répertoriées dans la cohorte prospective. Le logiciel pourrait ainsi fournir des scores de risques corrélés aux différents traitements identifiés dans la cohorte.

Un médecin face à une patiente en pré ou post ménopause pourrait ainsi fournir en données d'entrée au logiciel les variables personnelles de la patiente correspondant aux paramètres personnels de la cohorte. Le logiciel fournirait alors des scores de risques pour différentes pathologies dans le cas où la patiente ne prendrait aucun traitement hormonal substitutif (THS) ou préventif de l'ostéoporose et dans le cas où elle prendrait tel ou tel traitement. Une sortie du logiciel pourrait avoir la forme du tableau II suivant - les chiffres indiqués sont des exemples de présentation de résultats et ne proviennent pas d'un cas de simulation réel.

**Tableau II**

| Risque à 10 ans | Sans Traite ment | Prise d'œstrogène | Prise de progestérone micronisé | Prise de progestérone synthétique | SERM |
|---|---|---|---|---|---|
| Cancer du sein | 7% | 8% | 6% | 10% | 4% |
| Cancer de l'ovaire | 1% | | | | |
| Cancer du côlon | 1% | | | | |
| Ostéoporose | 5% | | | | |
| Cardiovasculaire | 15% | | | | |
| Alzheimer | 12% | | | | |

Le médecin pourrait ainsi mesurer le bénéfice et le risque de la prise de tel ou tel traitement en fonction des paramètres personnels de sa patiente et choisir ou non de prendre un THS ou un SERM (Sélective Oestrogène Receptor Modulators) et si oui lequel.

Les scores de risques fournis par un tel logiciel seraient calculés par la méthode, c'est-à-dire en estimant tout d'abord un module de scores de risques relatif individuel pour chacune des pathologies listées dans la première colonne du tableau II, puis en multipliant chaque score de risques du module par l'incidence de chaque pathologie dans la population à laquelle appartient la patiente si elle appartient à une population différente de celle de la cohorte prospective utilisée.

A partir de la cohorte prospective sélectionnée, la méthode propose de considérer si l'individu donné appartient à la même population que cette cohorte prospective. Si tel est le cas, une estimation des risques associés à telle ou telle pathologie peut se faire par une recherche de voisins dans la cohorte prospective ou par une méthode paramétrique comme le modèle de Cox. En revanche, si l'individu donné n'appartient pas à la même population que la cohorte prospective sélectionnée, un score estimé dans la cohorte prospective peut conduire à une sous estimation ou à une surestimation des risques liés à cette pathologie, comme expliqué en référence au tableau I. Une estimation d'un risque relatif avant multiplication par l'incidence de la pathologie dans la population à laquelle appartient l'individu à traiter sera alors nécessaire.

La méthode propose donc d'estimer tout d'abord un module de scores de risques relatif et individuel, par exemple en effectuant une recherche de voisins sur la cohorte prospective.

On entend par voisinage d'un individu donné, l'ensemble des individus de la cohorte qui présentent sensiblement les mêmes paramètres personnels. On définit alors une distance D entre l'individu donné I et un individu C de la cohorte, par exemple en calculant la somme des carrés des écarts entre chaque paramètre individuel de l'individu I et d'un membre de la cohorte C. On peut antérieurement appliquer un filtre pour exclure certains paramètres, individuels ou thérapeutiques, que l'on sait être insignifiants pour la variable pathologique à déterminer. Par exemple, si l'on souhaite estimer les risques de cancer du sein, on peut exclure les paramètres personnels de cholestérol. On peut aussi attribuer des poids différents aux différents paramètres individuels et thérapeutiques retenus. Une fois les distances D calculées entre l'individu I et les membres de la cohorte C, on fixe un seuil limite Dₗᵢₘ en deçà duquel un membre de la cohorte C sera considéré comme un voisin V. On peut ensuite pondérer l'importance des voisins en fonction de leur distance à l'individu donné I.

L'estimation du score de risques absolu individuel lié à une pathologie donnée pour un individu donné I pourra alors être une moyenne pondérée de la variable pathologie sur l'ensemble des voisins V de la cohorte. Une telle estimation de score de risques peut être effectuée pour chaque pathologie à examiner pour l'individu donné I. L'estimation des scores des risques pour différentes pathologies peut se faire à partir du même voisinage ; ou le voisinage peut être recalculé pour chaque variable pathologie à expliciter en appliquant des filtres différents d'exclusion de certains paramètres individuels ou thérapeutiques. On obtient ainsi un module de scores de risques représentatif des risques liés à différentes pathologies susceptibles d'être développées par l'individu I encore sain ; ce module de scores de risques absolus devient relatif en divisant par les risques moyen au même âge dans la cohorte et individuel par rapport aux paramètres de l'individu I donné.

Le module de scores de risques relatif individuel pourrait aussi être déterminé par une méthode paramétrique comme le modèle de Cox. Une fois les risques relatifs βi estimés, pour chaque paramètre explicatif individuel vi de la cohorte, on construit un risque relatif de moyenne proche de 1 en écrivant exp[βi(vi - valeur moyenne de vi)]. Il faut donc aussi estimer sur la cohorte prospective ces valeurs moyennes. Si on a plusieurs variables explicatives , ces exponentiels se multiplient pour donner un module de risque relatif de moyenne proche de 1 sur la population de la cohorte.

Le calcul du module de scores de risques relatifs, qu'il soit estimé par la méthode des voisins ou par le modèle de Cox, est effectué selon la même méthode quel que soit le nombre de paramètres et de variables considérés dans la cohorte. De ce fait, de nouveaux paramètres ou variables peuvent être intégrés dans une cohorte existante et dans un score existant sans changer fondamentalement la méthode de calcul. Cette flexibilité permet de rajouter des variables à un score de risque simplement en ajoutant au score un terme du type exp[βi(vi - valeur moyenne de vi)]. Comme ce terme est de moyenne proche de 1, les seuils précédents établis pour le score (ex 10%) restent valides en ajoutant ce nouveau terme. Ceci permet de garder un score au cours du temps, en l'enrichissant progressivement de variables épidémiologiques, biologiques ou même génétiques. Cette souplesse permet donc de s'adapter au mieux à la fois à la diversité des pays et aussi aux dernières recherches.

Comme indiqué précédemment, si l'individu donné I appartient à la même population que la cohorte, le module de scores de risques absolus estimé par la méthode des voisins constitue une bonne estimation des risques qu'encourt cet individu de développer telle ou telle pathologie. Néanmoins, si ledit individu I appartient à une autre population, les scores de risques déterminés comme indiqué ci-dessus sont biaisés, comme le montre le tableau I discuté précédemment. Le module de scores de risques à utiliser doit être un module relatif en divisant par les incidences moyennes dans la cohorte. La méthode propose donc de multiplier chaque score du module de risques relatif individuel par l'incidence de la pathologie dans la population à laquelle appartient l'individu I donné. L'incidence de chaque pathologie peut être déterminée comme le rapport du nombre d'individus ayant développés annuellement ladite pathologie dans la population finale sur le nombre total d'individus dans ladite population pour une même tranche d'âge.

On obtient ainsi un risque absolu annuel. Pour un risque à 10 ans d'une femme de 50 ans, on multipliera le risque relatif par la somme des incidences à 50 ans, à 51 ans ... jusqu'à 60 ans si on a des incidences par age précises à l'année. En général, on dispose de valeurs d'incidences par fourchettes d'ages. On multipliera alors le risque relatif par la somme des incidences par tranches d'âges.

Si dans la population finale on dispose seulement de l'incidence moyenne tous âges confondus, ce qui est le cas pour la majorité des pays, on obtiendra une estimation de l'incidence par âge en prenant un pays proche où cette courbe est connue et en mutlipliant les valeurs par l'incidence moyenne tous âges confondus du pays final divisée par celle de ce pays proche. On constate en effet que cette courbe d'incidence pour le cancer su sein est sensiblement différente pour les trois pays cités en exemple plus haut, France, Etats-Unis, Japon. Pour un pays asiatique par exemple, il faudra se baser plutôt sur la courbe du Japon, corrigée du rapport moyen d'incidence tous âges confondus de ce pays asiatique divisée par celle du Japon.

L'incidence de la pathologie dans la population finale peut être appliquée aux scores de risques relatifs car les risques relatifs ont été calculés à partir d'individus de la population initiale qui présentent les mêmes facteurs de risque que l'individu considéré. Le module de risque relatif est relativement uniforme d'un pays à l'autre, car l'effet d'un facteur de risque est sensiblement le même quelque soit le pays considéré. C'est ce qui justifie les « méta-analyse » en épidémiologie, regroupant des analyses sur plusieurs pays ou cohortes pour faire ressortir des risques relatifs ou « Odds Ratio » communs, ces Odds Ratio correspondent aux exp(βi). On peut avoir une autre illustration de cette constance des effets des facteurs de risque par exemple en cardiologie avec Yusuf S, et al. « Effect of potentially modifiable risk factors associated with myocardial infarction in 52 countries (the INTERHEART study): case-control study Lancet » , 2004 Sep 11-17;364 (9438):937-52.

A la différence de ces termes, le risque « de base » ou l'incidence moyenne ne sont pas uniformes selon les pays, il faut donc bien un procédé qui permette de séparer ce qui doit être exporté (le risque relatif individuel) et ce qui doit être pris dans le pays de la population finale (l'incidence moyenne).

Les variables explicatives peuvent avoir une différence de moyenne selon la population. Si leur moyenne est connue dans la population finale, c'est celle-ci qu'il faut prendre, sinon il faut prendre celles de pays considérés comme les plus proches en terme de risque et à défaut en rester aux moyennes connues sur la population initiale.

La méthode de l'invention peut être mise en œuvre par ordinateur. Un logiciel distribué aux médecins peut présenter une interface grâce à laquelle le médecin saisit un certain nombre de paramètres individuels propres à son patient et sélectionne les variables pathologiques qu'il souhaite déterminer. Le logiciel fournira un module de scores de risques comprenant une série de scores de risques associés à une série de pathologies sélectionnées par le médecin.

Le médecin peut sélectionner une option de population si le patient appartient à une autre population que la population dont est issue la cohorte prospective utilisée pour programmer le logiciel ; le médecin peut ainsi cocher la population à laquelle appartient son patient parmi un choix de populations proposé. Le logiciel pondérera alors le module de scores de risques relatif individuel par les incidences de chaque pathologie sélectionnée par le médecin pour fournir un module de scores de risques absolu.

Une autre option peut aussi être sélectionnée si le médecin envisage de prescrire à son patient tel ou tel traitement thérapeutique ; le médecin peut cocher une ou plusieurs cases correspondant aux traitements thérapeutiques envisagés. Le logiciel calculera alors un module de scores de risques relatif individuel sous forme matricielle comprenant une série de scores de risques associée aux pathologies sélectionnées par le médecin et des séries de scores de risques associées auxdites pathologies avec prise de tel ou tel traitement thérapeutique, comme montré dans le tableau II.

Selon un mode de réalisation, le logiciel peut être programmé pour calculer le risque d'une femme sous traitement particulier, par exemple sous estrogènes, en considérant que c'est une variable comme une autre. Selon un autre mode de réalisation, le logiciel calcule un risque sans traitement qui sera ensuite multiplié par le risque relatif dû aux estrogènes, par exemple 1,4 si on utilise les données des risques relatifs présentés dans l'article de Fournier et al, « Breast cancer risk in relation to différent types of hormone replacement therapy in the E3N-EPIC cohort », Int J Cancer. 2005 Apr 10;114(3):448-54. La publication retenue pour connaître le risque relatif d'un traitement doit être choisie pour que sa population d'étude soit proche de la population finale. On entend par risque relatif d'un traitement le risque d'une population sous traitement par comparaison à une population sans traitement ; ce risque risque relatif de traitement n'est pas à confondre avec le risque relatif individuel définit précedemment.

La méthode de l'invention permet donc une estimation plus fiable des scores de risques de pathologies pour des individus appartenant à des populations différentes de la population initiale de la cohorte prospective et permet aussi d'estimer l'impact de traitements thérapeutiques sur ces scores de risques.

## Revendications

1. Une méthode de détermination de scores de risques de pathologies pour un individu donné appartenant à une population finale, la méthode comprenant les étapes de :
- sélection d'une cohorte prospective à partir d'une population initiale différente de la population finale ;
- estimation d'un module de scores de risques relatif individuel de l'individu à partir de paramètres et de variables de la cohorte prospective, ledit module comprenant au moins un score de risques associé à au moins une variable pathologie ;
**caractérisée en ce que** la méthode comprend les étapes de :
- multiplication de chaque score de risques du module relatif individuel par l'incidence de chaque pathologie dans la population finale de l'individu ;
dans laquelle l'estimation d'un module de scores de risques relatif individuel comprend pour chaque pathologie les étapes de :
- estimation de risques relatifs (βi) pour chaque paramètre explicatif individuel (vi) de la cohorte et considéré comme explicatif de cette pathologie ;
- construction d'un risque relatif pour chaque paramètre explicatif exprimé comme exp[βi (vi - valeur moyenne de vi)] ;
- construction du module de scores de risques relatif individuel en multipliant les risques relatifs pour chaque variable pathologie donnée.

2. La méthode de la revendication 1, dans laquelle la cohorte prospective comprend :
- une sélection d'individus appartenant à une population initiale ;
- un répertoire de paramètres individuels pour chaque individu ; et
- un répertoire de variables pathologies pour chaque individu de la cohorte ayant développé des pathologies données.

3. La méthode de la revendication 2, dans laquelle la cohorte prospective comprend en outre des paramètres thérapeutiques pour chaque individu de la cohorte associés à la prise de traitement thérapeutiques par ledit individu.

4. La méthode de la revendication 3, dans laquelle les paramètres thérapeutiques comprennent les traitements hormonaux substitutifs ou des traitements préventifs de l'ostéoporose.

5. La méthode de l'une des revendications 1 à 4, dans laquelle le module de scores de risques relatif individuel de l'individu est multiplié par un risque relatif dû à un traitement donné.

6. Un programme d'ordinateur mettant en oeuvre la méthode de l'une des revendications 1 à 5, comprenant une interface permettant la saisie de paramètres individuels associés à un individu donné et la sélection d'au moins une variable pathologique à estimer, ledit programme fournissant un module de scores de risques relatifs individuels associés à ladite variable pathologique pour ledit individu.

7. Le programme de la revendication 6, dans lequel l'interface permet la saisie de la population finale à laquelle appartient l'individu donné, ledit programme fournissant un module de scores de risques absolu associés à ladite variable pathologique pour ledit individu.

8. Le programme de la revendication 6 ou 7, dans lequel l'interface permet la saisie d'au moins un paramètre thérapeutique associé à la prise d'un traitement thérapeutique, ledit programme fournissant un module de scores de risques matriciel comprenant au moins un score de risques associé à la variable pathologie sélectionnée et au moins un score de risque associé à ladite variable pathologie avec prise dudit traitement thérapeutique.

## Patentansprüche

1. Methode zur Bestimmung von Werten von Erkrankungsrisiken für eine gegebene Person, die einer Zielpopulation angehört, wobei die Methode die folgenden Schritte umfasst:
- Auswahl einer Studienkohorte aus einer Ausgangspopulation, die von der Zielpopulation verschieden ist,
- Abschätzung eines persönlichen, relativen Risikowertmoduls der Person aus Parametern und Variablen der Studienkohorte, wobei das genannte Modul mindestens einen Risikowert in Bezug auf mindestens eine Erkrankungsvariable umfasst,
**dadurch gekennzeichnet, dass** die Methode die folgenden Schritte umfasst:
- Multiplikation jedes Risikowertes des persönlichen, relativen Risikowertmoduls mit der Inzidenz jeder Erkrankung in der Zielpopulation der Person,
wobei die Abschätzung eines persönlichen, relativen Risikowertmoduls für jede Erkrankung die folgenden Schritte umfasst:
- Abschätzung relativer Risiken (βi) für jeden persönlichen Erklärungsparameter (vi) der Kohorte, der als diese Erkrankung erklärend angesehen wird,
- Aufstellung eines relativen Risikos für jeden Erklärungsparameter, ausgedrückt durch exp[βi(vi - Mittelwert von vi)],
- Aufstellung des persönlichen, relativen Risikowertmoduls durch Multiplikation der relativen Risiken für jede gegebene Erkrankungsvariable.

2. Methode nach Patentanspruch 1, in der die Studienkohorte umfasst:
- eine Auswahl von Personen, die einer Ausgangspopulation angehören,
- eine Liste persönlicher Parameter für jede Person
- eine Liste von Erkrankungsvariablen für jede Person der Kohorte, die gegebene Erkrankungen erlitten haben.

3. Methode nach Patentanspruch 2, in der die Studienkohorte außerdem für jede Person der Kohorte Therapieparameter in Bezug auf die Anwendung therapeutischer Behandlungen bei der genannten Person umfasst.

4. Methode nach Patentanspruch 3, in der die Therapieparameter Hormonersatzbehandlungen oder Behandlungen zur Osteoporosevorbeugung umfassen.

5. Methode nach irgendeinem der Patentansprüche 1 bis 4, in der das persönliche, relative Risikowertmodul der Person mit einem relativen Risiko in Bezug auf eine gegebene Behandlung multipliziert wird.

6. Computerprogramm, das die Methode nach irgendeinem der Patentansprüche 1 bis 5 ausführt, eine Schnittstelle umfassend, die die Eingabe von persönlichen Parametern erlaubt, die zu einer gegebenen Person gehören, und die Auswahl mindestens einer abzuschätzenden Erkrankungsvariablen erlaubt, wobei das genannte Programm ein persönliches, relatives Risikowertmodul mit Bezug auf die genannte Erkrankungsvariable für die genannte Person liefert.

7. Programm nach Patentanspruch 6, in dem die Schnittstelle die Eingabe der Zielpopulation erlaubt, der die gegebene Person angehört, wobei das genannte Programm ein absolutes Risikowertmodul mit Bezug auf die genannte Erkrankungsvariable bei der genannten Person liefert.

8. Programm nach Patentanspruch 6 oder 7, in dem die Schnittstelle die Eingabe mindestens einen Therapieparameters in Bezug auf die Anwendung einer therapeutischen Behandlung erlaubt, wobei das genannte Programm ein Matrixmodul von Risikowerten liefert, das mindestens einen Risikowert in Bezug auf die gewählte Erkrankungsvariable und mindestens einen Risikowert in Bezug auf die gewählte Erkrankungsvariable bei Anwendung der genannten therapeutischen Behandlung umfasst.

## Claims

1. A method for determining pathology risk scores for a given individual belonging to a final population, the method comprising the steps of:
- selection of a prospective cohort from an initial population different from the final population;
- estimation of a module of individual relative risk scores of the individual from parameters and variables of the prospective cohort, said module comprising at least one risk score associated with at least one pathology variable;
**characterized in that** the method comprises the steps of:
- multiplication of each risk score of the individual relative module by the incidence of each pathology in the final population of the individual;
wherein the estimation of a module of individual relative risk scores comprises for each pathology the steps of:
- estimation of relative risks (βi) for each individual explanatory parameter (vi) of the cohort and considered as explanatory for this pathology;
- construction of a relative risk for each explanatory parameter expressed as exp[βi (vi - mean value of vi)];
- construction of the module of individual relative risk score by multiplying the relative risks for each given pathology variable.

2. The method of claim 1, wherein the prospective cohort comprises:
- a selection of individuals belonging to an initial population;
- a directory of individual parameters for each individual; and
- a directory of pathology variables for each individual of the cohort having developed given pathologies.

3. The method of claim 2, wherein the prospective cohort further comprises therapeutic parameters for each individual of the cohort associated with therapeutic treatment by said individual.

4. The method of claim 3, wherein the therapeutic parameters comprise hormone replacement therapy or preventive treatment of osteoporosis.

5. The method of one of claims 1 to 4, wherein the module of individual relative risk scores of the individual is multiplied by a relative risk due to a given treatment.

6. A computer program implementing the method of one of claims 1 to 5, comprising an interface allowing the entry of individual parameters associated with a given individual and the selection of at least one pathological variable to be estimated, said program providing a module of individual relative risk scores associated with said pathological variable for said individual.

7. The program of claim 6, wherein the interface allows the entry of the final population to which the given individual belongs, said program providing an absolute risk score module associated with said pathological variable for said individual.

8. The program of claim 6 or 7, wherein the interface allows the entry of at least one therapeutic parameter associated with a therapeutic treatment, said program providing a matrix risk score module comprising at least one risk score associated with the selected pathology variable and at least one risk score associated with said pathology variable with said therapeutic treatment.
